# EUROPEAN PATENT APPLICATION

(11) **EP 4 781 976 A1**
(43) Date of publication of application: **29.07.2026**
(21) Application number: 23952453.1
(22) Date of filing: 18.09.2023
(51) Int. Cl.: A61K 8/92, A61K 8/06, A61K 8/97

(54) **COSMETIC COMPOSITION FOR THE SCALP, USE AND METHOD FOR TREATING THE SCALP**

(71) Applicant: Natura Cosméticos S.A., 05106-000 São Paulo SP (BR)
(72) Inventor: ARANDAS MONTEIRO E SILVA, Silas, 05106-000 São Paulo - SP (BR); CARVALHÃES LAGO NOLD, Juliana, 05106-000 São Paulo - SP (BR); STURION, Éder, 05106-000 São Paulo - SP (BR); KEIKO ALVES WADA, Joeni, 05106-000 São Paulo - SP (BR); LIMA, Edjane, 05106-000 São Paulo - SP (BR); CAPELAS ROMEU, Clarissa, 05106-000 São Paulo + SP (BR)
(74) Representative: Simmons & Simmons
(86) International application number: PCT/BR2023/050310
(87) International publication number: WO 2025/059735

(57) **Abstract**

The present invention is related to a cosmetic composition, particularly in the form of a serum, for application to the scalp, comprising *Astrocaryum murumuru,* in an amount sufficient to promote benefits to the scalp and hair, as well as related uses and methods.

## Description

### FIELD OF THE INVENTION

The present invention is related to a cosmetic composition, particularly in the form of a serum, for application to the scalp, comprising *Astrocaryum murumuru*, in an amount sufficient to promote benefits to the scalp and hair, as well as related uses and methods.

### FUNDAMENTALS OF THE INVENTION

The scalp is the part of the skin where the highest density of hair, known as hair strands, is concentrated. The skin of the scalp has specific characteristics that differentiate it from the rest of the skin on the body. For example, it has an intermediate thickness, between the skin of the eyelid, which is the thinnest, and that of the nape region, which is the thickest. It is the site of approximately 150,000 hair follicles, each with a hair shaft and a sebaceous gland. It is, therefore, a site of intense metabolic activity in sebum production and in the activity of the hair cycle, encompassing the anagen, catagen, and telogen phases of the hair strands.

In general, changes to the scalp, whether due to lesions, flaking, or even diseases-which may be of genetic, metabolic, systemic, or infectious origin-can compromise even the quality of the hair.

It is expected that a poorly nourished scalp, with deficiencies in natural moisturizing and repair factors, may produce dull, rough, naturally more porous hair strands that require repair of the cortex and cuticle. In this context, the main complaints observed in hair are rough texture, dullness, fragility, that is, hair that requires softness, shine, and repair from the origin of the strands.

In addition to stress management recommendations, a balanced diet, and the use of regular massages to stimulate blood circulation, several products focused on the scalp have been developed.

In general, the available products are aimed at acting directly on the damaged hair fiber or are intended for application to the scalp to prevent hair loss. In these cases, these products generally use a complex combination of active ingredients, with most including actives of synthetic origin.

Furthermore, the particularities of the scalp make it unfeasible for classically known ingredients to provide benefits for the skin in general, whether of natural or synthetic origin, to be researched for the development of products for the scalp.

*Astrocaryum murumuru,* known as murumuru, has been researched for skin care products. For example, international publication WO14053038, by Natura Cosméticos S.A., addresses cosmetic and/or pharmaceutical compositions comprising a vegetable lipid, which provide benefits to the skin, as demonstrated by biological mechanisms associated with general skin treatment.

Patent reference CN102198064, of Tianjin Tiens Biolog Dev Co, presents a specific hair treatment composition and a method for its preparation, which specifically uses murumuru butter. It is noted that the reference correlates with hair treatment associated with dandruff, directly related only to the scalp microbiota.

Thus, there remains in the state of the art a need for new cosmetic compositions that can provide direct benefits to the scalp, such as revitalization, repair, and deep hydration of this characteristic skin region, as well as offer additional benefits to the hair, such as anti-hair loss action, anti-thinning, stimulation of growth, thicker strands, repair of the hair fibers including cortex and cuticles from their origin (that is, from root to tip), promoting more integral strands with uniform texture, softness, and shine from their origin.

### SUMMARY OF THE INVENTION

In a first aspect, the present invention is related to a cosmetic composition, particularly in the form of a serum, for application to the scalp, comprising *Astrocaryum murumuru*, in an amount sufficient to promote benefits to the scalp and hair.

In a second aspect, the present invention contemplates the use of *Astrocaryum murumuru* in the preparation of cosmetic compositions for the treatment of the scalp and hair strands.

In a third aspect, the present invention is related to a cosmetic treatment method which comprises applying a cosmetic composition comprising *Astrocaryum murumuru* to the scalp to promote benefits to the scalp and hair.

### BRIEF DESCRIPTION OF THE DRAWINGS

Figure 1 presents a comparison of the quantification of representative images of increased BMP4 synthesis in human skin fragments, incubated with the product according to the present invention for 72 hours. The measurement was performed using the *Image J* program, and the unit of quantification is *square pixel*.
Figure 2 (A) to (I) shows the results of atomic force microscopy. (A) to (C) present the results for zone 1, (D) to (F) for zone 2, and (G) to (I) for control, at t0, t90, and t150, respectively.

### DESCRIPTION OF THE INVENTION

The present invention is related to a cosmetic composition comprising *Astrocaryum murumuru* capable of providing benefits to the scalp and, consequently, to the hair.

The cosmetic composition according to the present invention, particularly provided in the form of a serum, comprises from about 0.01 to about 5% of *Astrocaryum murumuru*, relative to the total weight of the composition, preferably in the form of butter.

The direct benefits to the scalp and, consequently, to the hair strands are achieved by modulating genes involved in the formation and development of the hair follicle and fiber, as well as in hair protection against oxidative stress and in scalp health.

The cosmetic composition according to the present invention, particularly in the form of a serum, comprising *Astrocaryum murumuru*, preferably in the form of butter, provides healthier scalp and hair strands by modulating at least one of the genes BMP4, CASK, ITGB4 and/or SOD2. Thus, a further aspect of the present invention is a cosmetic composition according to the present invention comprising *Astrocaryum murumuru* to modulate at least one of the aforementioned genes BMP4, CASK, ITGB4 and/or SOD2, particularly the BMP4 gene.

The cosmetic composition according to the present invention promotes an increase of at least 90% in BMP4, which is responsible for cell growth and differentiation, as well as for inducing differentiation of keratinocytes in the bulb matrix and the formation of the hair follicle and fiber.

According to the present invention, *Astrocaryum murumuru*, preferably in the form of butter, has been shown to act in the region of the dermal papilla and bulb, inducing the differentiation of keratinocytes to improve the quality of the hair fiber, through gene modulation of BMP4 (increased expression), which represents the main marker for the mechanism of hair formation due to its characteristic modulation of the keratinocyte life cycle.

Furthermore, the cosmetic composition according to the present invention promotes at least a 74% increase in CASK, which acts in the development of the hair follicle, in the differentiation of keratinocytes, and in the formation of the hair follicle.

Furthermore, the cosmetic composition according to the present invention provides an increase of at least 216% in ITGB4, which is associated with the embryonic processes of follicle formation, as well as the maintenance and protection of the follicle (dermal-epidermal junction), indicating scalp health.

The cosmetic composition according to the present invention further provides an increase of at least 91% in SOD2, which is responsible for protection against external aggressions and oxidative stress, as well as protection of the hair.

This remarkable action in modulating genes associated with scalp mechanisms, particularly ITGB4, SOD2, BMP4, and CASK, provides comprehensive repair benefits for the scalp and hair fibers, deep scalp hydration, enabling repair of the cortex and cuticles from their origin (root to tip), and more intact hair fibers with uniform texture, softness, and shine from their origin.

The cosmetic composition according to the present invention is prepared with cosmetically acceptable excipients, particularly for the preservation of a serum composition.

In a second aspect, the present invention contemplates the use of *Astrocaryum murumuru*, preferably in the form of butter, in an amount ranging from about 0.01 to about 5%, relative to the total weight of the composition, in the preparation of cosmetic compositions for the treatment of the scalp and, consequently, the hair strands.

In a third aspect, the present invention is related to a cosmetic treatment method that consists of applying a cosmetic composition comprising *Astrocaryum murumuru* to the scalp to promote benefits to the scalp and hair.

The following examples, without imposing any limitation, the particular embodiments of the present invention, as well as demonstrate that the cosmetic composition according to the present invention:
- activates the hair reconstruction power from its origin;
- promotes strands that are more repaired from the cuticle to the cortex since their origin;
- deeply hydrates the scalp;
- intensifies the production of shielded strands that are more resistant to breakage;
- provides natural shielding of the hair strands;
- protects and enhances keratin production;
- provides a healthier cuticle and cortex, prepared to withstand the daily damage to which the hair fibers are exposed;
- results in strands with shine and vitality from their origin;
- stimulates the formation of strands that are more resistant to daily damage;
- promotes hair with more mass (keratin quality), shielded cuticles, and less susceptible to breakage;
- stimulates the formation of high-quality keratin for greater resistance to daily damage, that is, hair with more mass, shielded cuticles, and less susceptible to breakage;
- stimulates the formation of a strand with a high-quality structure for resistance to daily damage, that is, hair with more mass, shielded cuticles, and less susceptible to breakage;
- stimulates the renewal of the scalp and follicles, restoring the vitality and softness of the scalp and hair strands, so that the scalp is healthier and protected for shinier, more manageable hair;
- promotes comprehensive reconstruction, both of the scalp and the hair strands, restoring the health of the hair, allowing its formation with more intact and prepared cuticles and cortex to withstand external damage;
- restores the hair's natural shine;
- activates the self-shielding of the strands, promoting strands that are more resistant to physical, chemical, and thermal damage;
- activates the hair reconstruction power from their origin, in order to restore the hair strands and the hair bulb, promoting the growth of healthier and stronger strands;
- provides hair that is deeply restored from the inside out;
- results in hair with a more uniform, healthy, and shiny texture;
- promotes the restoration and reconstruction of the hair strands from the root;
- results in strands of higher quality, that is, strands with less porosity from their formation;
- the strands grow shinier, softer, and more uniform;
- Improves cuticle texture from the root; and
- provides nourishment from root to tip, that is, enhanced nutrition from the origin of the strands.

Based on the evaluation of the following tests, it is possible to state that the use of the composition according to the present invention is capable of improving scalp health and impacting the formation of new hair strands. Improved quality keratin formation and reduced hair fiber roughness were observed. These benefits have as their mechanism of action primarily the modulation of BMP4 (a gene related to follicle development and hair fiber formation.)

### EXAMPLES

### Example 1A - Serum composition according to the present invention

| Ingredient | (%) |
|---|---|
| Water | 70.861 |
| Alcohol | 13.89 |
| PEG-40 Hydrogenated Castor Oil | 6 |
| Glycerin | 5 |
| Propanediol | 1.998 |
| Murumuru seed butter *Astrocaryum murumuru* | 1 |
| Fragrance | 0.5 |
| Hydroxyacetophenone | 0.3 |
| Citric acid | 0.3 |
| Sodium gluconate | 0.1 |
| Tocopherol | 0.05 |
| Linalool | 0.022525 |
| Benzyl salicylate | 0.0225225 |
| Limonene | 0.0088825 |
| Coumarin | 0.0045045 |
| Sodium hydroxide | 0.000993677 |
| Sodium carbonate | 0.000006022 |
| Sodium chloride | 0.000000301 |

### Example 1B - Serum composition according to the present invention

| Ingredient | % |
|---|---|
| Water | q.s. |
| Alcohol | 10 |
| PEG-40 Hydrogenated Castor Oil | 3 |
| Glycerin | 1 |
| Propanediol | 0.5 |
| Murumuru seed butter *Astrocaryum murumuru* | 0.01 |
| Fragrance | 0.25 |
| Hydroxyacetophenone | 0.10 |
| Citric acid | 0.10 |
| Sodium gluconate | 0.01 |
| Tocopherol | 0.01 |
| Linalool | 0.005 |
| Benzyl salicylate | 0.005 |
| Limonene | 0.001 |
| Coumarin | 0.001 |
| Sodium hydroxide | 0.005 |
| Sodium carbonate | 0.005 |
| Sodium chloride | 0.005 |

### Example 1C - Serum composition according to the present invention

| Ingredient | % |
|---|---|
| Water | q.s. |
| Alcohol | 20 |
| PEG-40 Hydrogenated Castor Oil | 10 |
| Glycerin | 10 |
| Propanediol | 10 |
| Murumuru seed butter *Astrocaryum murumuru* | 5 |
| Fragrance | 1.5 |
| Hydroxyacetophenone | 0.5 |
| Citric acid | 1.0 |
| Sodium gluconate | 0.5 |
| Tocopherol | 1.0 |
| Linalool | 0.10 |
| Benzyl salicylate | 0.10 |
| Limonene | 0.10 |
| Coumarin | 0.10 |
| Sodium hydroxide | 0.10 |
| Sodium carbonate | 0.10 |
| Sodium chloride | 0.10 |

### Example 2 - Ex vivo evaluation of BMP4 protein synthesis

Bone morphogenetic proteins (BMPs) comprise a group of more than 20 proteins belonging to the transforming growth factor (TGF)-β superfamily. Among their functions, they stand out as powerful regulators of skin development and as controllers of epidermal homeostasis, hair follicle growth, and melanogenesis.

In the context of melanogenesis, bone morphogenetic protein 4 (BMP4) can act as an autocrine inhibitor of melanogenesis. The BMP-4 effect is mediated by transcriptional and post-transcriptional events, leading to partial repression of the tyrosinase promoter, as well as decreased stability and levels of tyrosinase mRNA. With regard to skin aging, BMP4 also plays a fundamental role in tissue maintenance. The protein is closely linked to the process of skin stem cell proliferation and is absolutely necessary for proper differentiation into various lineages of fully differentiated skin cells. Once inappropriate biological activities of skin stem cells become inducers of wrinkle formation, BMPs acting appropriately tend to inhibit this undesirable process.

In this study, the ability of the composition according to the present invention (as per example 1) on the synthesis of BMP4 in human skin fragments was evaluated.

### Incubation of skin fragments ex vivo

After the surgical procedure performed by the ophthalmic plastic surgery medical team, eyelid fragments were placed in vials containing 0.9% saline solution until the time of the experiment. Biological samples were removed from the saline solution, immersed in 70% ethanol for 15 seconds, and washed twice in fresh saline solution. The fragments were transferred to a petri dish containing culture medium for treatment with the composition according to the present invention, for 72 hours, in a humid atmosphere, 37 °C, CO₂ at 5%. The product was applied to the skin at a rate of 2 mg/cm².

### Immunofluorescence evaluations

After 72 hours of incubation with the composition according to the present invention, the *ex vivo* skin fragments were fixed in 4% paraformaldehyde (pH 7.4) for 24 hours and cryoprotected in 30% sucrose solution for 48 hours. Next, serial sections of 10 µm were collected directly onto silanized slides with the aid of a cryostat (Leica - CN1850).

At the end of the section collection, the sections were washed with 0.1 M PB and incubated *overnight* with the primary antibody BMP4 (Abcam, ab39973). Subsequently, they were subjected to additional washes with 0.1 M PB and incubated for 1 hour with secondary antibody *Goat anti-mouse* (Invitrogen; A11055). At the end of these steps, a new incubation (1 minute) with DAPI (4'-6-Diamidino-2-Phenylindole; DNA marker) was performed, followed by 3 washes of 5 minutes each with 0.1 M PB.

Next, the slides were mounted in a specific mounting medium and analyzed under a fluorescence microscope (Leica - DM 1000) with the aid of the LAS Software (Leica Application Suite). The parameter evaluated was the intensity of fluorescence emitted by labeling with a specific antibody.

### Data analysis

Analysis of variance (ANOVA) was used for statistical analysis. The *Paired t test* was used when the analysis of variance detected significant differences between the groups. In all groups studied, those with P values less than 0.05 were considered statistically significant.

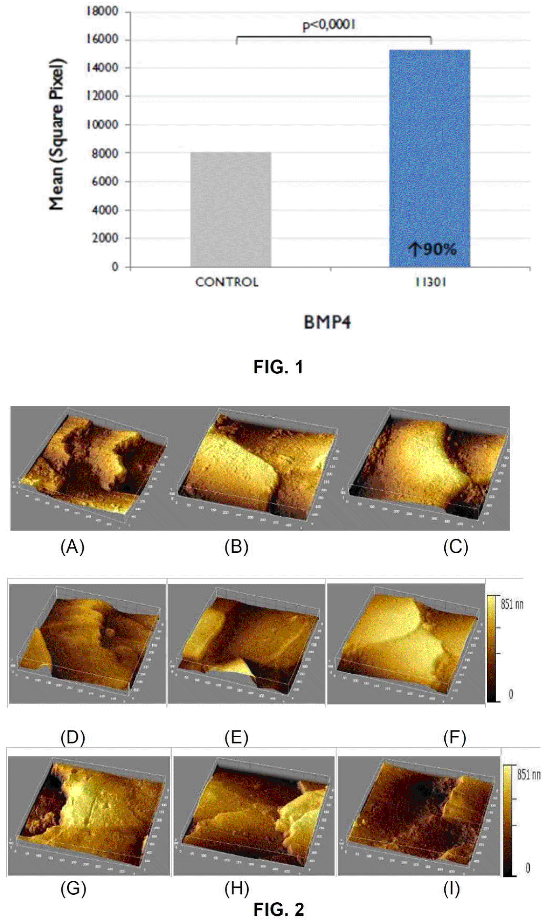

Figure 1 presents the results of BMP4 synthesis measurement in human skin fragments from 3 different patients, treated with the composition according to the present invention for 72 hours, compared to the control (untreated) under the same conditions. When compared to the control, the composition according to the present invention showed a positive modulatory effect on BMP4 synthesis, promoting a 90% increase.

According to the results presented, it is concluded that the composition according to the present invention demonstrated the ability to positively modulate BMP4 synthesis in human skin fragments, when compared to the control group, promoting a 90% increase.

### Example 3 - Scalp irritation test

For the present study, nineteen patients completed the study. Average age: 45 ± 12 years; Phototype: 89% phototype III and 11% phototype IV; Gender: 89% female and 11% male.

The study was conducted over approximately five months.

After recruitment, patients were instructed to discontinue the use of any topical products on the scalp and hair up to 48 hours before the start of the study. On the day of the study, the patients who attended the laboratory received explanations from the researcher about the study procedures, legal and ethical aspects, risks and benefits, medical support, and methods of reimbursement for participation expenses. The methodology of subjective analysis by clinical efficacy was conducted by the dermatologist at the beginning of the study and after 90 and 150 days of use of the investigational product.

Daily, the patient applied the composition according to the present invention over the entire scalp at night, massaging with the fingertips to distribute it well and facilitate its penetration. It was requested not to rinse and to let it dry.

According to the study protocol and procedures used to evaluate the efficacy of the product under investigation, it was possible to verify that the composition according to the present invention reduced erythema in 53% of the research subjects after 150 days of use.

There was no variation in the parameters of irritation, burning sensation, and itching. This indicated that the composition according to the present invention did not cause these symptoms and protected the scalp, preventing the appearance of any signs or symptoms of sensitivity and/or irritation.

The composition according to the present invention reduced scalp flaking in 32% of the research subjects after 150 days of use.

Regarding erythema, it was observed that after 90 days of use of the composition according to the present invention, there was a 3.1% reduction in erythema, with a responsiveness of 5%, and after 150 days there was a 3.1% reduction in erythema, with a responsiveness of 53%.

Regarding scaling, some of the participants presented mild to moderate scaling, and after 90 and 150 days the condition improved. After 90 days of use of the composition according to the present invention, there was a 37.5% improvement in desquamation, with a responsiveness of 16%, and after 150 days there was an 87.5% improvement in flocculation, with a responsiveness of 32%.

From these results, it is possible to conclude that the composition according to the present invention contributes to the improvement of scalp health, as it is capable of reducing desquamation.

### Example 4 - hair fiber test

The objective of this study was to evaluate cuticle lifting and hair fiber roughness after the application of the composition according to the present invention using atomic force microscopy technique.

Five research patients completed the study. Average age: 48 ± 11 years; Phototype: 100% phototype III; Gender: 100% female.

The study was conducted over approximately five months.

After recruitment, patients were instructed to discontinue the use of any topical products on the scalp and hair up to 48 hours before the start of the study. On the day of the study, the patients who attended the laboratory received explanations from the researcher about the study procedures, legal and ethical aspects, risks and benefits, medical support, and methods of reimbursement for participation expenses.

For the evaluation of hair roughness, strands were collected from the root and three regions of the strands were analyzed by atomic force microscopy: Zone 1 - Keratinization Zone, Zone 2 - 1.5 cm of hair shaft growth, and Zone 3 - mid-length (control region).

Daily, apply to the entire scalp at night, massaging with your fingertips to distribute well and facilitate product penetration. Do not rinse and allow to dry. Application once daily was requested.

According to the protocol and procedures used in this study, it was possible to conclude that in zone 2 (1.5 cm of hair shaft growth), after 90 and 150 days, the strands showed significantly (P<0.05) lower roughness values when compared to the strands in the initial condition. Furthermore, after 90 days, the composition according to the present invention showed an improvement in roughness values of 14.5%, reaching 24.2%, and after 150 days showed an improvement in roughness values of 21.4%, reaching 35.3%.

In zone 1 (keratinization zone), after 90 and 150 days, the fibers showed significantly (P<0.05) lower roughness values compared to the fibers in the initial condition. Furthermore, after 90 days, the experimental product showed an improvement in roughness values of 11.3%, reaching 17.3%, and after 150 days, it showed an improvement in roughness values of 12.5%, reaching 19.5%.

The comparison between zone 2 (1.5 cm of hair shaft growth) and zone 3 (control region) showed a significant reduction in the roughness values of zone 2 when compared to zone 3 (control) after 90 and 150 days of home use.

Surprisingly, the composition according to the present invention showed an improvement in fiber roughness as determined by atomic force microscopy.

Roughness is an important attribute for the surface and assesses the mechanical/structural perspective of the fiber; in this case, it refers to the newly formed fiber, not a modification resulting from deposition on the fiber, but rather from genetic/protein modulation in the scalp, leading to the formation of a healthier fiber.

This evaluation was carried out based on the assessment of different parts of the strand.

In zone 1 (keratinization zone or growth zone), there was an improvement in roughness, making it possible to observe a more uniform shape of hair fiber, which in the future will result in a strand with less roughness. The results are illustrated in Figure 2 (A), (B), and (C), where after 90 days there was an 11% improvement in roughness, which can reach 17.3%, and after 150 days there was a 12% improvement in roughness, which can reach 19.5%.

In zone 2 (distal region - proximal to the bulb), the keratin is already fully formed, making it the best region for assessing roughness. The results are presented in figure 2 (C) to (E), where it was observed that after 90 days there was an improvement of 14.5% in roughness, which can reach 24.2%, and after 150 days there was an improvement of 21.4% in roughness, which can reach 35.3%.

In the control, shown in figure 2 (F) to (I), where there is no possibility of action by the composition according to the present invention, the roughness remained, as it is very far from the hair bulb.

Comparing zone 2 with the control zone, the results of zone 2 show statistical significance (P<0.05).

### Example 5 - hair integrity by means of differential scanning calorimetry (DSC).

The objective of this study was to evaluate the protection of the α-keratin structure of the hair fiber through DSC (differential scanning calorimetry).

Twenty research patients completed the study. Average age: 43 ± 12 years; Phototype: 75% phototype III and 25% phototype IV; Gender: 100% female.

The study lasted for about five months.

The hair fibers were randomly collected for DSC analysis at the initial condition (t0), and after 150 days of product use (t150). Thermal analyses were performed in triplicate by DSC, totaling 3 data sets per treatment.

Daily, the sample was applied over the entire scalp at night, massaging with the fingertips to distribute well and facilitate penetration. The product was applied once a day.

According to the results, when comparing the degradation enthalpy data of α-keratin (deltaHD) in the initial condition and after 150 days of product use, respectively (for each research subject), after 150 days of product use, 90% of the subjects showed an increase in the degradation enthalpy of α-keratin (deltaHD) compared to the hair fibers in the initial condition (t0). After 150 days of product use, 10% of the patients showed no significant differences in the degradation enthalpy values of α-keratin (deltaHD) compared to the hair fibers in the initial condition (t0).

According to the results, when comparing the degradation temperature data of α-keratin (TD) in the initial condition and after 150 days of product use, respectively (for each research subject), it was observed that after 150 days of product use, 30% of the subjects showed an increase in the degradation temperature of α-keratin (deltaD) compared to the hair fibers in the initial state (t0). After 150 days of product use, 70% of the subjects showed no significant differences in the degradation temperature (TD) of α-keratin compared to the hair fibers in the initial condition (t0).

The objective of this test was to evaluate the protection of the alpha-keratin structure in the hair fiber based on the amount of energy required to break the structure.

No cosmetic composition capable of improving the quality of newly formed keratin has been identified in the state of the art. In general, scalp care products refer to overall care, involving factors such as flaking, redensification (increasing the number of strands), and combating hair loss.

It was therefore found that after 150 days, 90% of the participants showed an increase in the variation of energy required for keratin degradation, which means that the keratin formed has better quality compared to T0.

The person skilled in the art will readily be able to assess, through the teachings contained in the text and the examples presented, the advantages of the invention and propose equivalent variations and alternatives of implementation, without departing from the scope of the invention as defined in the appended claims.

## Claims

1. COSMETIC COMPOSITION FOR THE SCALP **characterized by** comprising *Astrocaryum murumuru* and cosmetically acceptable excipients.

2. COMPOSITION, according to claim 1, **characterized by** comprising from about 0.01 to about 5% of *Astrocaryum murumuru* relative to the total weight of the composition.

3. COMPOSITION, according to claim 2, **characterized by** *Astrocaryum murumuru* being in the form of butter.

4. COMPOSITION, according to any one of claims 1 to 3, **characterized by** being in the form of a serum.

5. COMPOSITION, according to any one of claims 1 to 4, **characterized by** modulating at least one of said genes BMP4, CASK, ITGB4 and/or SOD2.

6. COMPOSITION, according to claim 5, **characterized by** modulating the BMP4 gene.

7. COMPOSITION, according to any one of claims 1 to 4, **characterized by** increasing BMP4 modulation by at least 90%, CASK modulation by at least 74%, ITGB4 modulation by at least 216%, and/or SOD2 modulation by at least 91%.

8. USE of the composition as defined in any one of claims 1 to 7, **characterized by** being for promoting complete repair of the scalp and hair strands, deep hydration of the scalp, repair of the cortex and cuticles from their origin, hair strands with greater integrity, with uniform texture, softness, and shine from their origin.

9. USE of *Astrocaryum murumuru* **characterized by** being in the preparation of a cosmetic composition for scalp treatment.

10. USE, according to claim 9, **characterized by** being useful in the complete repair of the scalp and hair strands, deep hydration of the scalp, repair of the cortex and cuticles from their origin, hair strands with greater integrity, with uniform texture, softness, and shine from their origin.

11. USE, according to claim 9, **characterized by** the fact that the amount of *Astrocaryum murumuru* ranges from about 0.01 to about 5% relative to the total weight of the composition.

12. METHOD FOR SCALP TREATMENT **characterized by** comprising applying a cosmetic composition comprising *Astrocaryum murumuru* to the patient in need.
